# EUROPEAN PATENT APPLICATION

(11) **EP 1 840 212 A1**
(43) Date of publication of application: **03.10.2007**
(21) Application number: 05703831.7
(22) Date of filing: 19.01.2005
(51) Int. Cl.: C12N 15/00, C12N 15/11, C12Q 1/68, G01N 33/50, G01N 33/15

(54) **METHOD OF SYSTEMATIC ANALYSIS OF RELEVANT GENE IN RELEVANT GENOME REGION (INCLUDING RELEVANT GENE/RELEVANT HAPLOTYPE)**

(71) Applicant: GENESYS TECHNOLOGIES, INC., Chuo-ku, Tokyo 103-0023 (JP)
(72) Inventor: TANAKA, Junji, Kamakura-shi, Kanagawa 247-0071 (JP)
(74) Representative: Möbus, Steffen
(86) International application number: PCT/JP2005/000594
(87) International publication number: WO 2006/077631

(57) **Abstract**

It is intended to provide a systematic analysis method wherein the virtual haplotype of a gene polymorphism serving as a marker is assumed and then a relevant haplotype block and a gene or a genome domain relating to a phenotype are successively determined from the whole genome domain or a genome domain of interest. As FIG. 1 shows, a discontinuous analysis method of the embodiment 1 comprises repeating the steps of constructing a virtual block from a discontinuous genome domain, determining a haplotype block based on a virtual haplotype, and then analyzing the relevancy to thereby determine a genome domain relating to a phenotype. Thus, it is possible to determine a relevant haplotype block, a relevant haplotype and a relevant gene.

## Description

### BACKGROUND OF THE INVENTION

### Field of the Invention:

This invention relates to a method of systematic analysis of relevant gene to specify a single or a plurality of relevant genome domain (including relevant gene /relevant haplotype) relating to a phenotype of disease susceptibility, drug responsiveness, or the like from the whole genome domain (or a discontinuous search genome domain).

### Description of the Related Art:

In the conventional analysis of relevant gene to specify relevant gene (or relevant genome domain) to be a marker of gene polymorphism represented by the microsatellite or the SNP (Single Nucleotide Polymorphism: a single nucleotide polymorphism or a position of single nucleotide polymorphism) relating to the realization of tailor made medicine such as disease susceptibility, drug responsiveness, or the like, the gene polymorphism marker typing by a wet process (Note 1) was performed after specifying genome domain to be analyzed by the particular genetical knowledge and narrowing down the gene polymorphism marker to be desired to analyze to several tens or several thousands of locations due to cost. This genome domain to be analyzed is mainly ccDNA domain or exon domain, and the known gene polymorphism marker to be typed is mainly in the domain.

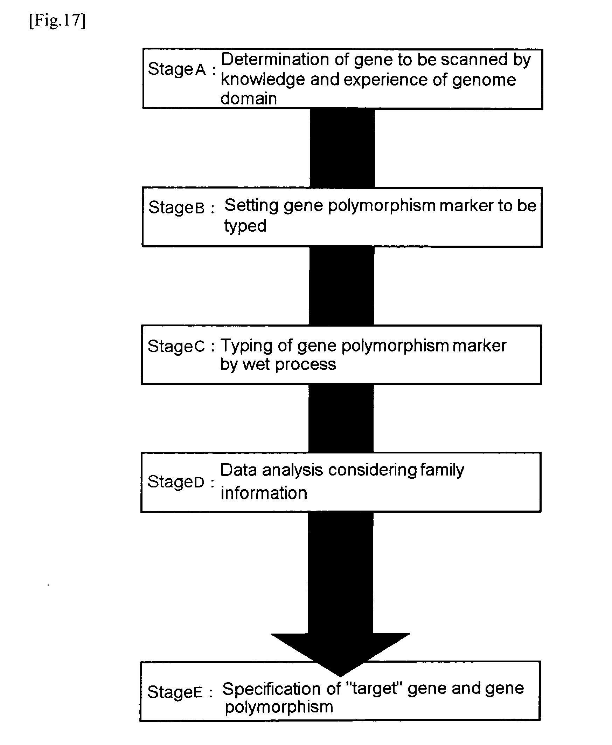

Fig. 17 is a drawing showing the process flow of the conventional analysis of relevant gene. As shown in Fig. 17, the conventional analysis of relevant gene is performed in the order of stage A (determining of gene or genome domain to be investigated), preliminary stage B (setting gene polymorphism markers to be typed), stage C (typing gene polymorphism markers by a wet process), stage D (analyzing data) and stage E (specifying the 'target' gene).

In the normal relevant gene analysis process, the gene polymorphism markers to be typed (hereafter referred to as 'typing gene polymorphisms') are limited, and function analysis is performed after narrowing down the gene polymorphisms to at most about 10, 000.

However, there is no other method to determine whether or not there is a relationship between disease susceptibility or drug responsiveness and gene polymorphism markers and relevant gene than by statistical determination from the results of typing those gene polymorphism markers. Therefore, 'target' genes (Note 2) /'target' haplotypes (Note 3), which are finally determined to be related, must be included in and selected from a group of 1,000 to 10,000 gene polymorphism markers preliminary as typing SNP. In the case that these gene polymorphism markers are not selected, the related gene polymorphism cannot be found in the analysis, and so the analysis process must be performed again from selection of a group of typing gene polymorphism.

In the conventional method of selecting typing gene polymorphism and relevant gene, the researcher used a technique of searching reference documents such as research papers or the like and genome-related databases or the like, and performing a homological search or the like that predicts the function of human genes that are similar to genomes that are not human whose functions area already known. Therefore, in most cases, this method is limited in exon domain or cDNA domain.

However, the functions of human genomes are not completely given in this genome information. Therefore, the step of selecting typing SNP that determine the efficiency of this SNP function analysis process, or in other words, whether or not it is possible to predict a 'target' SNP at a high probability, depends largely on the experience and skill of the researcher as well as luck.

Furthermore, relevant gene / relevant haplotype of multifactorial disease exists in the discontinuous domain frequently, but it is impossible to link between gene (polymorphism) / haplotype which exists in the discontinuous domain and phenotype in conventional method. Especially when a specific combination of a plurality of gene (polymorphism) / haplotype are related to phenotype, it is difficult to specify their relationship.

Taking the aforementioned problems into consideration, the object of the present invention is to provide a systematic specifying method that the gene polymorphisms that may contain polymorphism having unknown function are selected and combined from discontinuous domain and comprise a virtual block, then a relevant haplotype block is narrowed down from the virtual block by using associated analysis or the like, and then target gene / genome domain and combination thereof are effectively specified by using associated analysis or the like for the haplotype frequency in the haplotype block.

(Note 1) The wet process is a process for performing SNP typing. Statistical analysis of the specified typing data is not included in the wet process.

(Note 2) The 'target' genes or genes that will become the 'target' means either two genes that are the cause of a phenotype which is considered to research the relationship between the genes and disease susceptibility, drug responsiveness (of newly developed drugs), or the like, or that are the indicators of a phenotype which is considered to research the relationship between genes and disease susceptibility, drug responsiveness, or the like. The object of the gene function analysis is to specify these genes.

(Note 3) The 'target' haplotypes or haplotypes that will become the 'target' means either two haplotypes that are the cause of a phenotype which is considered to research the relationship between the haplotypes and disease susceptibility, drug responsiveness (of newly developed drugs), or the like, or that are the indicators of a phenotype which is considered to research the relationship between haplotypes and disease susceptibility, drug responsiveness, or the like. The object of the haplotype function analysis is to specify these haplotypes.

### SUMMARY OF THE INVENTION

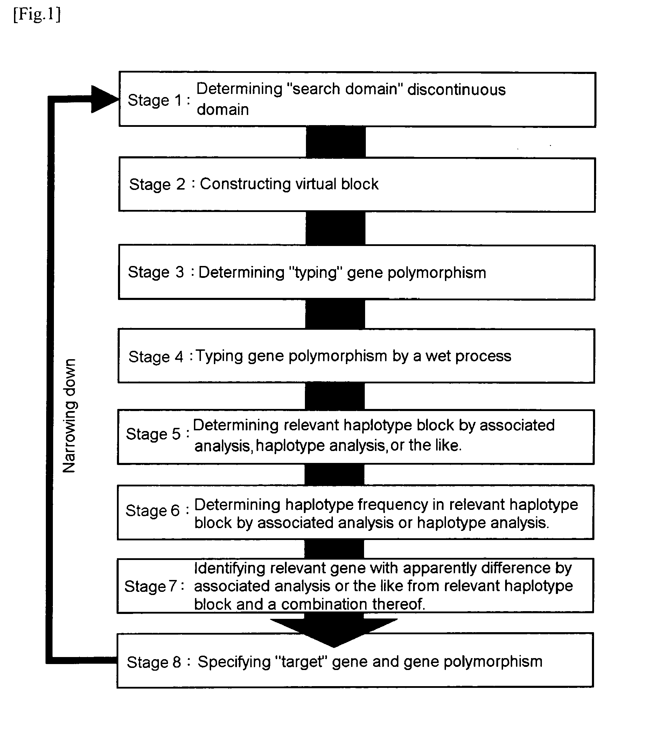

The invention according to claim 1 is a method of systematic analysis of relevant gene identifying relevant genome domain of a single or a plurality of relevant gene / relevant haplotype or the like related to disease susceptibility, drug responsiveness, or the like (abbreviation afterward as 'relevant genome domain') from the information about a whole genome domain or a discontinuous genome domain which can be included in a part of function to be desired to analyze is elucidated or is not all estimated (abbreviation afterward 'search domain') and comprising: a first step of constructing a virtual block from a whole genome domain or a combination of a part of discontinuous genome domain (stage 2 in Fig. 1); a second step of specifying haplotype block (or genome domain) by scanning said virtual block using virtual haplotype (stage 5 in Fig. 1); a third step of calculating haplotype frequency by associated analysis in said haplotype block (or genome domain) (stage 6 in Fig. 1); a fourth step of specifying haplotype block / haplotype and combination thereof which has an apparent difference by associated analysis (stage 7 in Fig. 1); and a fifth step of identifying said relevant gene / regevant haplotype and a combination thereof from said haplotype block (stage 8 in Fig. 1).

The invention according to claim 2 is a method of systematic analysis of relevant gene identifying relevant genome domain of a single or a plurality of relevant gene / relevant haplotype or the like related to disease susceptibility, drug responsiveness, or the like (abbreviation afterward as 'relevant genome domain') from the information about a whole genome domain or a discontinuous genome domain which can be included in a part of function to be desired to analyze is elucidated or is not all estimated (abbreviation afterward 'search domain') and comprising: a first step of constructing a virtual block from said search domain; a second step of specifying haplotype block by scanning said virtual block using virtual haplotype; a third step of calculating haplotype frequency by haplotype analysis, associated analysis, or the like in said haplotype block; a fourth step of specifying haplotype block which has an apparent difference by said haplotype analysis, associated analysis, or the like; and a fifth step of identifying said relevant genome domain from said haplotype block.

The invention according to claim 2 is the method of systematic analysis of relevant gene according to claim 1 in which said first step comprises a step of selecting a known marker for every haplotype block when the marker representing the each haplotype block is known, and making the continuous virtual block by connecting over the marker.

The invention according to claim 3 is the method of systematic analysis of relevant gene according to claim 1 in which said first, second, third, fourth, and fifth step comprises a step of determining the gene polymorphism marker to specify said relevant genome domain and gradually (including one step) narrowing down said relevant genome domain by repeating all the steps or a part of step.

The invention according to claim 4 is the method of systematic analysis of relevant gene according to claim 1 in which said second step comprises a step of determining a single or a plurality of haplotype block which is linkage disequilibrium (or chains) state to said relevant genome domain using statistical analysis such as virtual haplotype analysis or the like

The invention according to claim 5 is the method of systematic analysis of relevant gene according to claim 1 in which said third step comprises a step of calculating the maximum likelihood origin haplotype and the frequency thereof by using the combination of associated analysis, haplotype analysis, and the like.

The invention according to claim 6 is the method of systematic analysis of relevant gene according to claim 1 in which said fourth step comprises a step of identifying said haplotype block including haplotype which has an apparent difference by said associated analysis.

The invention according to claim 7 is the method of systematic analysis of relevant gene according to claim 1 in which said second step comprises a step of determining the border of said haplotype block by using statistical data such as the number of combination of virtual haplotype, entropy value, the number of said maximum likelihood origin haplotype, linkage disequilibrium value, or the like.

The invention according to claim 8 is the method of systematic analysis of relevant gene according to claim 1 in which said third step comprises a step of determining a group of said maximum likelihood origin haplotype by using EM algorithm, MCMC method, or the like.

The invention according to claim 9 is the method of systematic analysis of relevant gene according to claim 1 in which said fourth step comprises a step of comparing a calculated statistical amount by associated analysis or the like with a preset or measured reference statistical amount, and when there is significant deviation between said statistical amount and said reference statistical amount that exceeds a preset threshold, determining said relevant genome domain is included in the domain (haplotype block) corresponding to the deviated position that exceeds said threshold value.

The invention according to claim 10 is the method of systematic analysis of relevant gene according to claim 1 in which said fifth step comprises a step of further detailed scanning / analyzing haplotype block or haplotype which has an apparent difference by said associated analysis or the like using sequencing or the like and determining said relevant genome domain.

The invention according to claim 11 is the method of systematic analysis of relevant gene according to claim 1 in which said fifth step comprises a step of selecting typing gene polymorphism marker having an interval that is at least shorter than the length of haplotype block in said search genome domain and that is as uniform as possible.

The invention according to claim 12 is the method of systematic analysis of relevant gene according to claim 1 in which said fifth step comprises a step of selecting the gene polymorphism marker with easy typing, which the gene polymorphism marker for typing in a group history expression is at least older than the phenotype which consider to check the relationship (SNP whose minor allele frequency is not so small number when the gene polymorphism is SNP) without limiting the cDNA domain or exon domain.

The invention according to claim 13 is the method of systematic analysis of relevant gene according to claim 1 in which said first, second, third, fourth, and fifth step comprises a step of determining the most preferred relevant haplotype block or relevant haplotype by changing the selecting method for virtual haplotype (length or the like).

The invention according to claim 14 is a computer program that can be read by a computer that can execute the processing of the method of specifying SNP according to any one of the claims 1 thru 13 wherein all of the steps of any one of the claims 1 thru 13 are coded.

The invention according to claim 15 is a system of analysis of discontinuous domain identifying relevant genome domain of a single or a plurality of relevant gene /relevant haplotype or the like related to disease susceptibility, drug responsiveness, or the like from the information about the search domain as a whole genome domain or a discontinuous genome domain which can be included in a part of function to be desired to analyze is elucidated or is not all estimated and comprising: a means of constructing a virtual block from said search domain; a first means of specifying haplotype block by scanning said virtual block using virtual haplotype; a means of calculating haplotype frequency by haplotype analysis, associated analysis, or the like in said haplotype block; a second means of specifying haplotype block which has an apparent difference by said haplotype analysis, associated analysis, or the like; and a means of identifying said relevant genome domain from said haplotype block.

### DESCRIPTION OF THE PREFERRED EMBODIMENTS

The preferred embodiments of the present invention are explained below based on the drawings.

### (Embodiment)

A virtual block defines as all the genome domain or a virtual continuous domain that made by connecting some discontinuous domains in all the genome domain as described above.

A haplotype block is a linkage disequilibrium state and a DNA domain to be inherited as a block which the history of recombination is hardly observed.

A virtual haplotype connects the gene polymorphism information about a part of genome domain regardless of whether or not there is a chain (a linkage disequilibrium) in 'search domain' and indicates the domain that is considered the combination or the combination thereof.

'A relevant genome domain' can also be regarded as the causative gene of the phenotype whether or not drug responsiveness, whether or not the particular disease, or the like directly and the identifying domain to identify whether or not there is a phenotype such as haplotypes which contains the causative gene directly, and this domain can also be regarded as a continuous or discontinuous domain. The virtual haplotype analysis indicates the analysis by using a virtual haplotype. Furthermore, 'maximum likelihood origin haplotype' indicates the plausible haplotype for explaining the haplotype in the group when the haplotype phase of the individual in the group is not specified

Fig. 1 is an example to show a brief summary of an analysis flowchart relating to the systematic specifying method of 'relevant genome domain' in the first embodiment of the present invention. As shown in Fig. 1, the systematic specifying method of 'relevant genome domain' in this embodiment comprises: a step (stage 1) of determining the search domain, a step (stage 2) of constructing the virtual block, a step (stage 3) of determining 'typing' gene polymorphism, a step (stage 4) of typing gene polymorphism by a wet process, a step (stage 5) of determining the 'relevant genome domain' by statistical analysis such as associated analysis, haplotype analysis, or the like, a step (stage 6) of determining the haplotype frequency by statistical analysis such as associated analysis, haplotype analysis, or the like in 'relevant genome domain', a step (stage 7) of identifying the relevant gene (or more specific relevant genome domain) which has an apparent difference from 'relevant genome domain', and a step (stage 8) of specifying the 'target' gene (target gene) and gene polymorphism, and wherein stage 1 to stage 7 can be repeated as one cycle. The present invention is that a wet process is not included in the present claims but included in the embodiment since the wet process is included in a part of the analytical flow when repeating it.
In this analytical method, by performing the described seven stage, 'relevant gene domain' related to a phenotype of disease susceptibility, drug responsiveness, or the like is (gradually) narrowed down from the 'search domain' to perform typing of the gene polymorphism marker initially, and 'relevant genome domain' related to a phenotype of whether or not drug responsiveness for the newly developed drug or the like is specified finally. This 'relevant gene domain' may be the case of polymorphism from one gene, the case of combination of polymorphism from a plurality of genes, or the case of one gene. When the 'relevant genome domain' is specified, the characteristics to be expressed are without assuming the particular knowledge about 'a related genome domain' and also without assuming the family line information of the sample.

Next, the process by each of the steps will be explained in detail with reference to Fig. 1.

(a) When specifying 'relevant gene domain' related to a phenotype of disease susceptibility, drug responsiveness, or the like, the analysis beginning from stage 1 is performed between two groups, which is a group of patients and a group of non-patients, a group of having effect or side effect and a group of having no effect, or the like. In this case, when the reference statistical amount is the whole of certain group, it is possible to compare with the general databases corresponding to the group.

(b) Stage 1 (determining the 'search domain'): In this analysis, by repeating the process from this stage 1 to stage 7 (to be explained later) as one cycle, the 'search domain' can be gradually narrowed down from an initially large 'search domain' to a more localized 'search domain'. In the case when the phenotype desired to be search has absolutely no information related to the genotype, it is preferred that the whole genome domain which includes the interesting domain other than exon, is set as the 'search domain'. In the case when the genome domain is known in the larger level such as a gene, a larger chromosome, or the like, in the case when the relevant genome domain is assumed from the particular genetic information, in the case when a plurality of chromosomes are the cause and it is not clearly known which chromosomes are suspect (including the 'relevant genome domain'), in the case when all the chromosomes except for certain chromosomes is the object (when there is no difference in the result between male and female, the sex chromosomes are meaningless so the measures are taken such as to remove them from the 'search domain'), or the like, the 'search domain' can be largely narrowed down. Moreover, more specifically, it is possible to set the initial 'search domain' at the gene level for example. In other words, the search domain (primary search domain, initial search domain) is set based on a chromosome level for which the functions are known in advance.

(c) Stage 2 is constructing the virtual block, which is virtually continuous, from the determined 'search domain' by connecting each continuous domain. This based virtual haplotype is treated as one continuous domain and the following analysis were performed.

(d) Determining the gene (polymorphism) typing from the virtual block. In the case when there is no gene polymorphism to be the clear candidate, it is preferred to select the gene polymorphism with easy typing (checking uniquely and easily) within the predicted gene polymorphism which the phenotype considered to check the relationship is formed at least older than thought to be expressed in a group history of the object in at least shorter interval than the haplotype block from the 'search domain'.

(e) Stage 4 is typing the gene polymorphism by a wet process, and the information about the typed gene polymorphism is preferred to confirm whether or not the correct object of gene polymorphism is typed by establishing Hardy-Weinberg Equilibrium or the like.

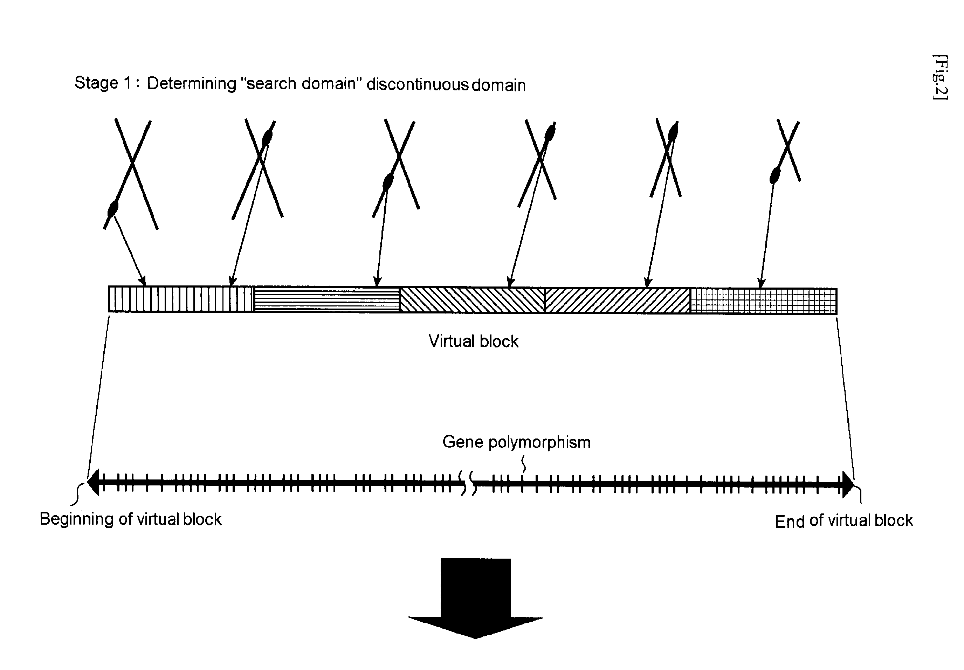

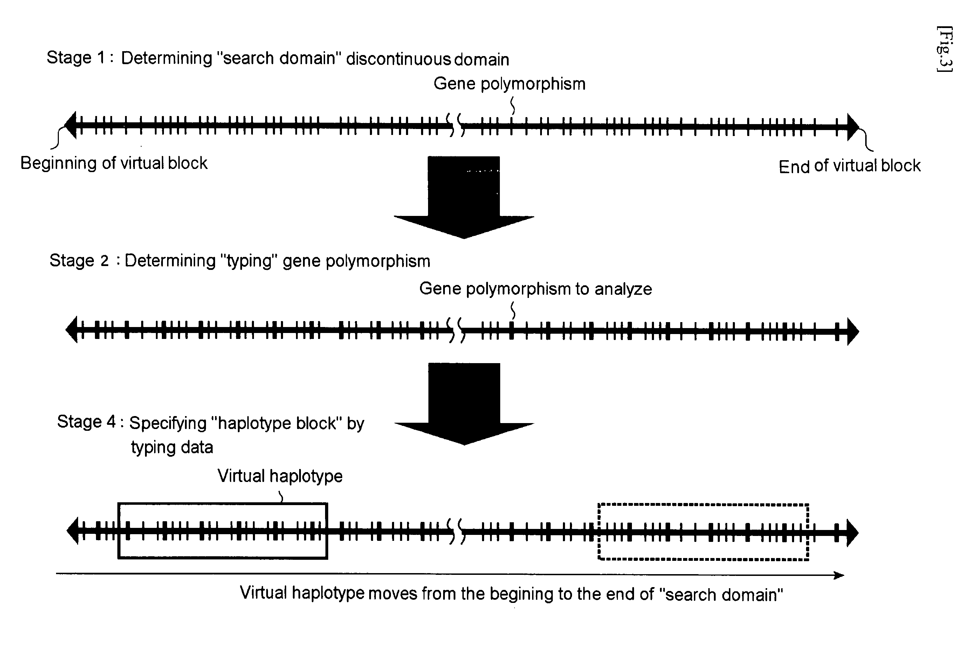

(f) Stage 5 is the stage for scanning the haplotype block in the typed gene polymorphism data by associated analysis, haplotype analysis, or the like, which is shown in Fig. 2, 3, and 4. Haplotype block is a chain of gene information (or a linkage disequilibrium state), which is treated as one information unit. Moreover, in the haplotype block, the kind of haplotype observed is several kinds in almost all groups.

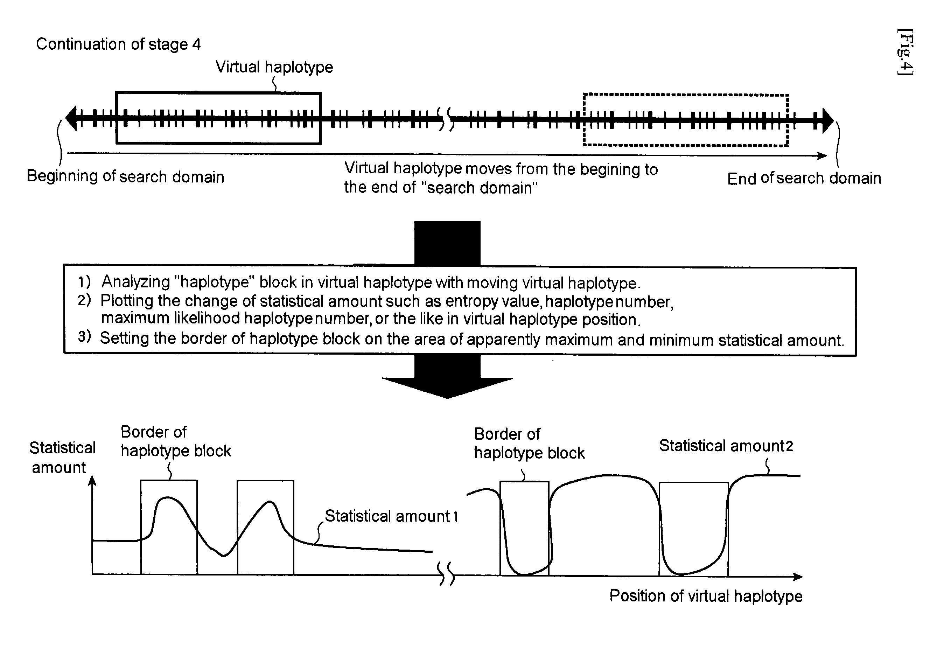

Hereby, as shown in Fig. 4, an apparent difference appears between the case in which the virtual haplotype was taken in the haplotype block and over the haplotypes. When the virtual haplotype is taken in the haplotype block, the kind of haplotype in a group is decreased, however, when the virtual haplotype is taken over the haplotype blocks, the kind of haplotype in a group is increased. It is clear when the phase of the haplotype is specified, it can be clear that the kind number of haplotype which can be taken in a group even when the phase is not specified, and even it can be also clear, by using EM algorithm, MCMC method, or the like by estimating the 'maximum likelihood origin haplotype' of the group when it is not appeared. It is beginning to demonstrate that the 'maximum likelihood origin haplotype' of the group estimated in the haplotype block conforms well to the haplotype when the phase is specified.

Since the kind of haplotype is limited in the haplotype block, when the virtual haplotype is taken in the haplotype block, the entropy of the haplotype calculated in a group becomes small (orderly), however, when the virtual haplotype is taken over the haplotype block, the entropy of the haplotype in a group becomes high (disorderly).

Besides this, since in the haplotype block since there is a linkage disequilibrium, when the various degrees of the linkage disequilibrium are used, the virtual haplotype in the haplotype block becomes the large degree of the linkage disequilibrium and the virtual haplotype over the haplotype block becomes the small degree of the linkage disequilibrium. Thus, it can be selected the haplotype block from the search domain and the genome domain which is considered to concentrate the information such as the haplotype block. When the haplotype block is not clearly determined, it can also be searched and determined by using the present analysis flow with detailed examination on the position which is likely to have the haplotype block.

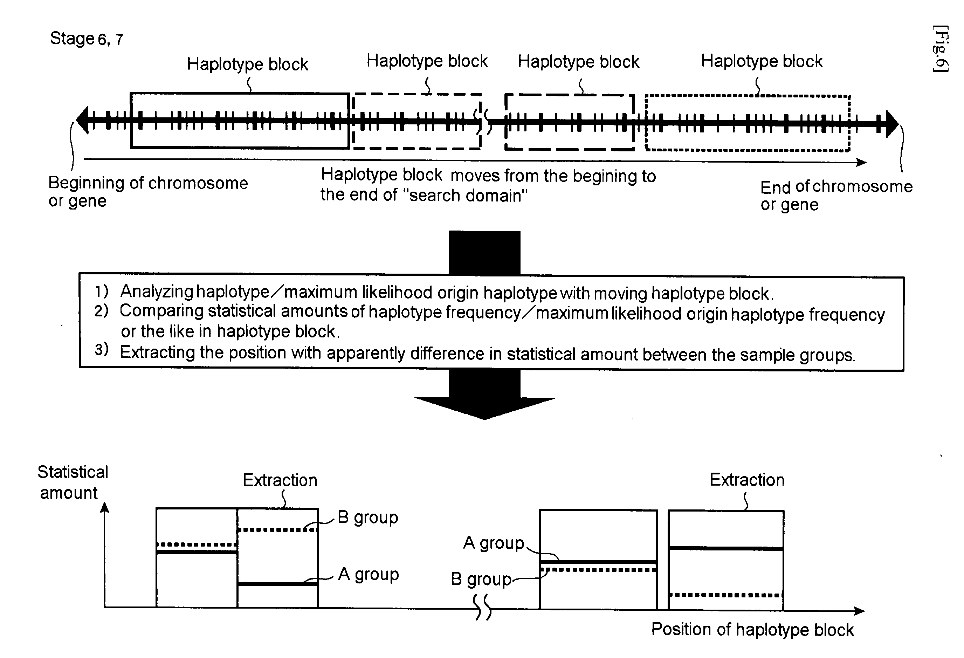

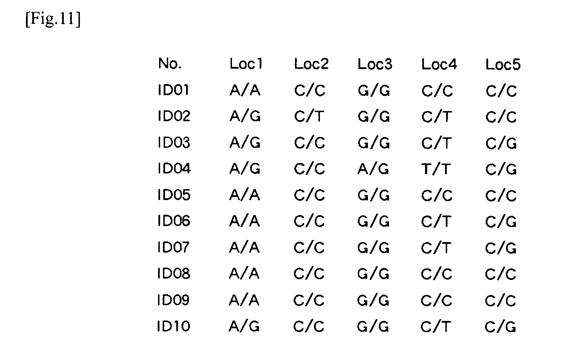

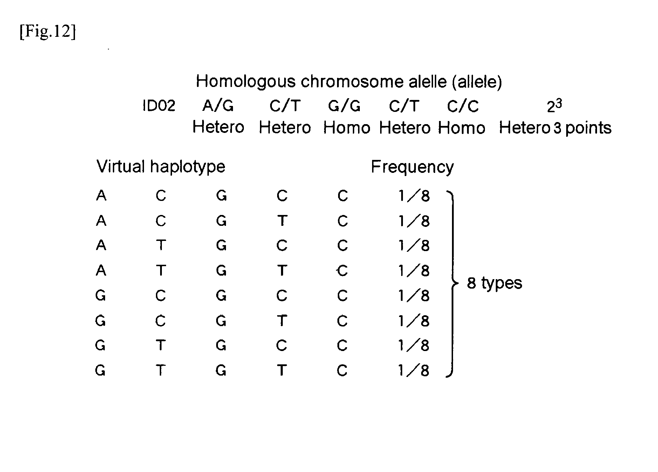

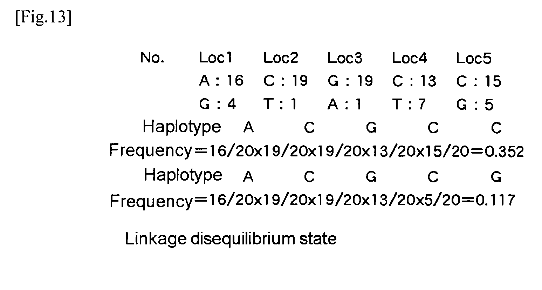

(g) Stage 6 is, as shown in Fig. 6, the process calculating the haplotype frequency in haplotype block. It is common that gene polymorphism information is obtained as shown in Fig. 11. This information shows that ID01 or the like indicates the sample number and Loc1 or the like indicates the position of the genome locus. The information of a homologous chromosome is shown in the data, and when the homology locus information is equivalent, it is called a homozygote, and when that is not equivalent, it is called a heterozygote. The statistical process is performed by the information as shown in this figure 11, and the information of the haplotype frequency can be calculated as shown in Fig. 13. The method calculating the haplotype frequency is described briefly (the gene polymorphisms are SNPs and the loci are five for simplification here). As shown in Fig. 12, the haplotype which is the combination of the allele (the allele of SNP) on one gamete (one part of the pair chromosome) is predicted stochastically when the phase is not specified. For example, the allele information of ID02 is shown below.

| | | | | | |
|---|---|---|---|---|---|
| ID02 | A/G | C/T | G/G | C/T | C/C |

As for predicting the haplotype stochastically, SNP#1 which takes A or G, SNP#2 which takes C or T, SNP#3 which takes G only, SNP#4 which takes C or T, and SNP#5 which takes C only, it is considered that the case of the haplotypes which are taken by five SNPs mentioned above. SNP#1, #2, and #4 are heterozygote and SNP#3 and #5 are homozygote.

| | Virtual haplotype | | | | | Frequency (Likelihood) |
|---|---|---|---|---|---|---|
| 1 | A | C | G | C | C | 1/8 |
| 2 | A | C | G | T | C | 1/8 |
| 3 | A | T | G | C | C | 1/8 |
| 4 | A | T | G | T | C | 1/8 |
| 5 | G | C | G | C | C | 1/8 |
| 6 | G | C | G | T | C | 1/8 |
| 7 | G | T | G | C | C | 1/8 |
| 8 | G | T | G | T | C | 1/8 |

This is the haplotype which sample ID02 can take and can be considered that each haplotype is the frequency (likelihood) 1/8 (=0.125).

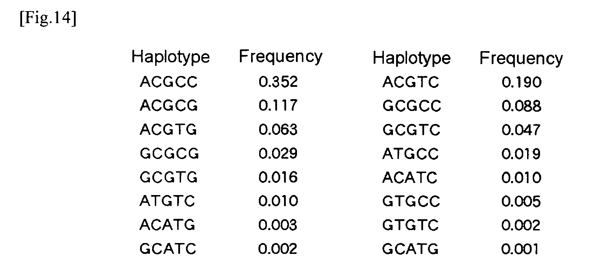

Such a counting is shown in Fig. 13. When it standardizes by adding to the sample 10, the likelihood of each haplotype can be calculated as shown in Fig. 14.

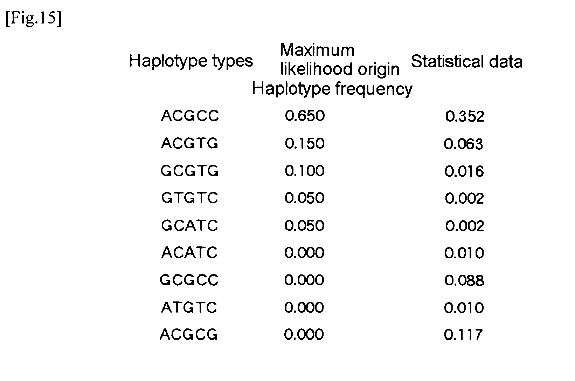

Thus, the data which was taken the statistics in a group can also be used as it is, the [maximum likelihood origin haplotype] of the group can be estimated by using the MCMC method, the EM algorithm, or the like from this statistical data, and the [maximum likelihood origin haplotype] can also be compared (reference to Fig. 15). When no linkage disequilibrium is observed between these SNPs, it is thought that the frequency of appearance of each SNP allele will become steady at an 'average' value, and since each of the SNPs is 'independent', the haplotypes that are statistically calculated will not be concentrated at a certain haplotype, but will be widely and thinly dispersed.

On the other hand, when linkage disequilibrium is observed between the analyzed SNP groups, then SNPs that statistically characterize the sample groups are contained in the groups, and the frequency of appearance of a certain allele in those SNPs increases. Also, it is predicted that the probability distribution of haplotypes that is the result of statistical analysis of these SNP data will concentrate on a certain haplotype (when assay is not performed for the 'target' SNP).

As the method of identifying the concentration at this certain haplotype, besides comparing the frequency of appearance of each individual haplotype, the total number of haplotypes predicted from that analytical data, the standard deviation of these haplotypes, and the ratio of the appearance frequency with respect to all of the haplotypes of the upper probability haplotype group are observed as the 'statistical amount,' and it can be identified by estimating the method of the 'maximum likelihood origin haplotype' or the like to be compared them by using the EM algorithm, the MCMC method, or the like between sample groups that are separated according to the expression of the characteristic of whether or not a drug is effective or has side effects.

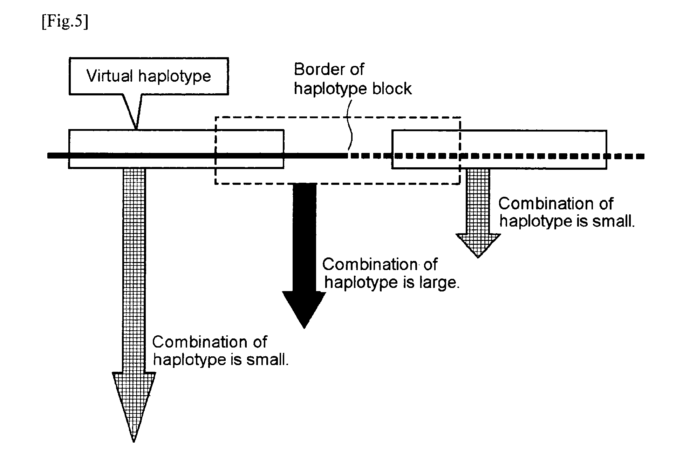

(h) Stage 7 is to identify the position which has an apparent difference between the groups which is desired to compared by using associated analysis, haplotype analysis, or the like (reference to Fig. 5 and 6). There can be a variety of cases about the group such as (patient / non-patient), (drug responsiveness positive / negative), (side effect positive / negative), or the like. By observing the apparent difference of haplotype frequency, which is calculated in stage 6 between these groups, the relationship to the relevant phenotype to be examined is identified. In the haplotype block, since the haplotypes are limited to the several kinds, the relevant haplotype block with the phenotype can be identified by comparing the several kinds of haplotypes.

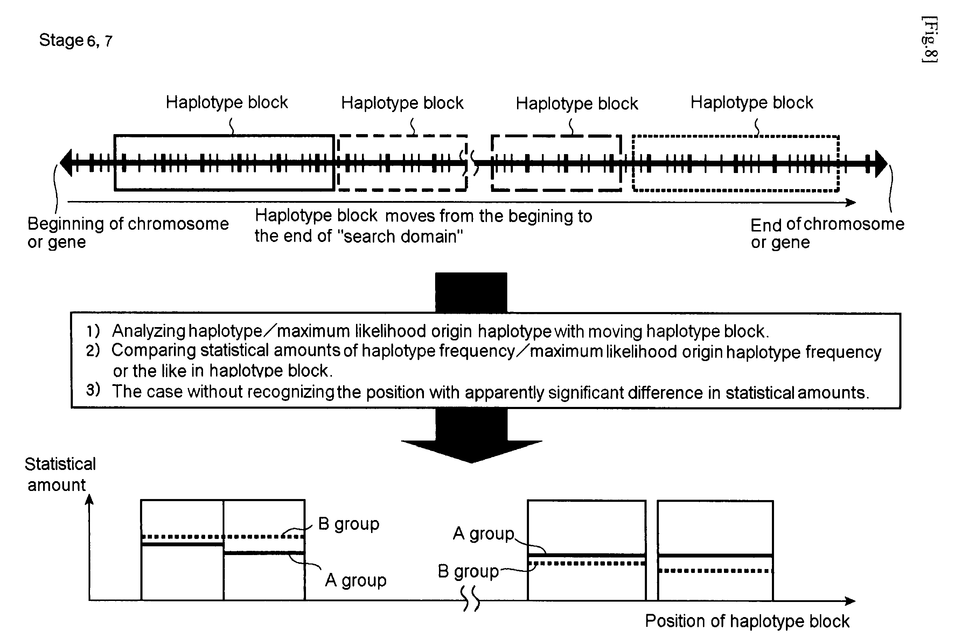

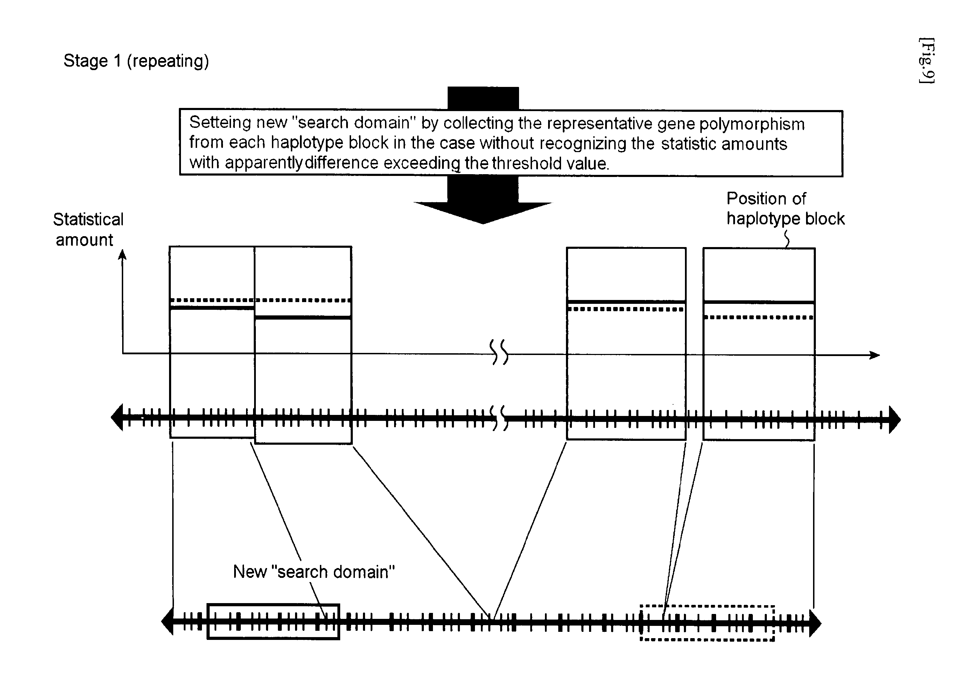

(i) In one haplotype block, it is considered that the case when the apparent difference is not observed between groups (Fig. 8). The apparent difference can be identified by constructing one virtual haplotype block with connecting the haplotype block, and by analyzing with considering that the whole virtual block is one virtual haplotype. This is a case when the specific combination of each haplotype block is related to the phenotype, and an example of identifying the phenotypes such as the nultifactorial disease or the like (Fig. 9).

(j) Stage 5, stage 6, and stage 7 can also be performed as a separate step, and can also be performed three steps simultaneously.

It is very difficult to select and directly type a 'target' SNP. This problem is solved by applying the linkage disequilibrium state between the 'target' gene (polymorphism) and the nearby gene polymorphism, and by estimating the domain near the 'target' gene polymorphism (haplotype block). The nearby gene polymorphism with the linkage disequilibrium state is expected that the probability distribution of the haplotype will change similarly when compared with the 'target' gene polymorphism is analyzed directly. This nearby gene polymorphism is considered to be a 'marker' gene polymorphism for the 'target' gene polymorphism. In other words, the statistical amount of the sample that is the object of analysis (the group with effect) is compared with the reference statistical amount of the reference sample (the group without effect) and when the difference exceeds a preset threshold value, it is determined that there was change in the corresponding typing domain (estimated as the marker gene polymorphism), and the corresponding search domain is set as a new search domain, and the next processing cycle is performed.

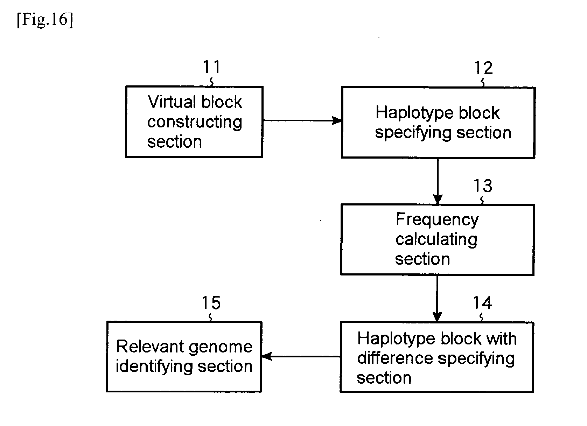

Fig. 17 shows a block diagram illustrating the brief summary of an example of the relevant genome domain specifying system in an embodiment of the present invention. The relevant genome domain specifying system can be constructed by a computer, and the relevant genome domain specifying system is constructed with the virtual block constructing section 11, the haplotype block specifying section 12, the frequency calculating section 13, the different haplotype specifying section 14, and the relevant genome identifying section 15 as shown in Fig. 16.

For example, the virtual block constructing section 11 constructs the virtual block from the search domain. The haplotype block specifying section 12 scans the virtual block and specifies the haplotype block by using the virtual haplotype. The frequency calculating section 13 calculates the haplotype frequency in the haplotype block by using haplotype analysis, associated analysis, or the like. The different haplotype specifying section 14 specifies the haplotype block which has an apparent difference by using haplotype analysis, associated analysis, or the like. The relevant genome identifying section 15 identifies the relevant genome domain from the specified haplotype block. The relevant genome domain specifying system constructed in this manner can perform the various processings as described above.

As described above, this 'related genome domain' specifying analysis does not always have to use the family information. When the family information is obtainable, it can be incorporated, however, the equivalent result can be obtained without the family information. Instead of the family information, the analysis is performed with the concepts such as haplotype block, associated analysis, virtual haplotype, or the like.

Finally the evaluation tests are performed by using Chi-square test or associated analysis to evaluate 'whether the expected results are obtained at a certain percent of probability', or 'whether severe side effects occur at a certain percent of probability', or the like, or the prediction can be shown how many times of the difference in the correlation intensity of the phenotype positive / negative with the genotype. Furthermore, by the haplotype block and the haplotype which can be judged the positive / negative phenotype, the selection of the 'relevant genome domain', and specification of the relevant gene polymorphism which is represented the relevant genome domain, the information of the concise realization of tailor made medicine can be provided.

The method of specifying the relevant gene of a first embodiment in the present invention is as described above, and it has the following effect.

When specifying the gene polymorphism from the typed gene polymorphism that is related to disease susceptibility, drug responsiveness, or the like, by narrowing down the base sequence domain that is the object of the analysis from a large domain to a more localized domain (haplotype block), and it is possible to finally specify these related genes (polymorphisms).

### Industrial Applicability

Since the present invention is constructed as described above, it has the following effects.

As explained above, with this invention, by estimating haplotyoe block (or the equivalent) from the gene polymorphism to be a marker, the base sequence domain that is the object of analysis is narrowed down from a large domain to a more localized domain (statistical amounts between the groups are compared and the relevant genome domain is narrowed down), and it is possible to finally specify genes (polymorphisms) related to the phenotype of disease susceptibility, drug responsiveness, or the like.

### BRIEF EXPLANATION OF THE DRAWINGS

[Fig.1] Fig. 1 is a brief summary showing an example of a process flowchart indicating the method of specifying 'relevant genome domain' of an embodiment of the present invention.
[Fig.2] Fig. 2 is a drawing showing an example of constructing the virtual block from the discontinuous genome domain in stage 2 of Fig. 1.
[Fig.3] Fig. 3 is a drawing showing an example of determining the border of haplotype block by using the virtual haplotype from virtual block in stage 1, 3, and 5 of Fig. 1.
[Fig.4] Fig. 4 is a drawing showing an example of determining the border of haplotype block by using the virtual haplotype from virtual block in stage 1, 3, and 5 of Fig. 1.
[Fig.5] Fig. 5 is a drawing showing an example of determining the border of haplotype block in stage 5 of Fig. 1 in detail.
[Fig.6] Fig. 6 shows an example of extracting the haplotype block which has an apparent difference by relevant analysis or the like between two groups in the haplotype block in stage 6 and 7 of Fig. 1.

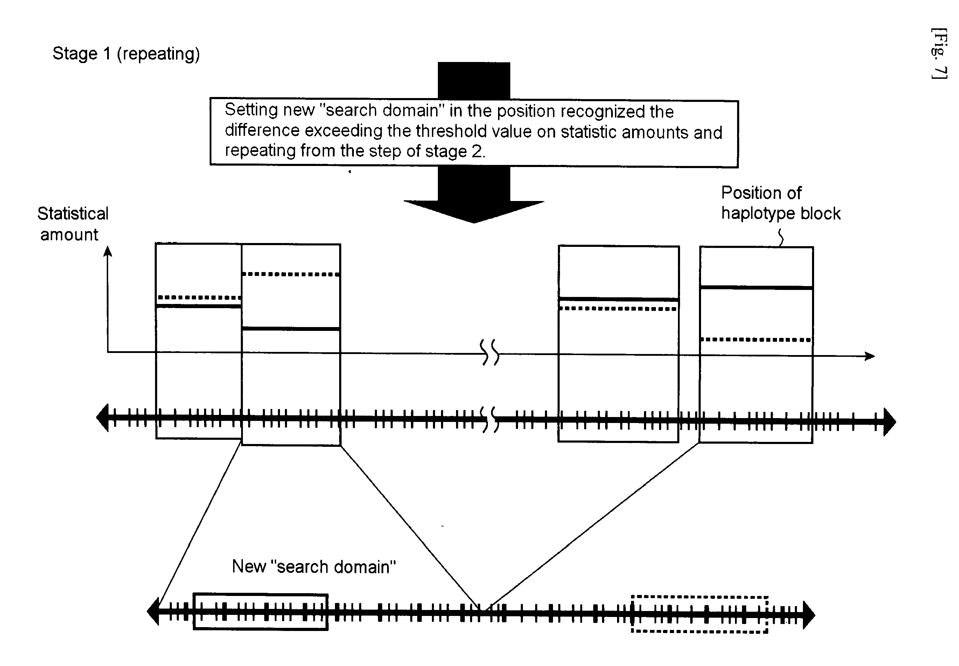

[Fig.7] Fig. 7 shows an example of constructing new virtual block from the haplotype block extracted in Fig. 6.
[Fig.8] Fig. 8 shows an example of constructing new virtual block when not extracting the haplotype block which has an apparent difference in stage 6 and 7 of Fig. 1.
[Fig.9] Fig. 9 shows an example of constructing new virtual block when not extracting the haplotype block which has an apparent difference in stage 6 and 7 of Fig. 1.

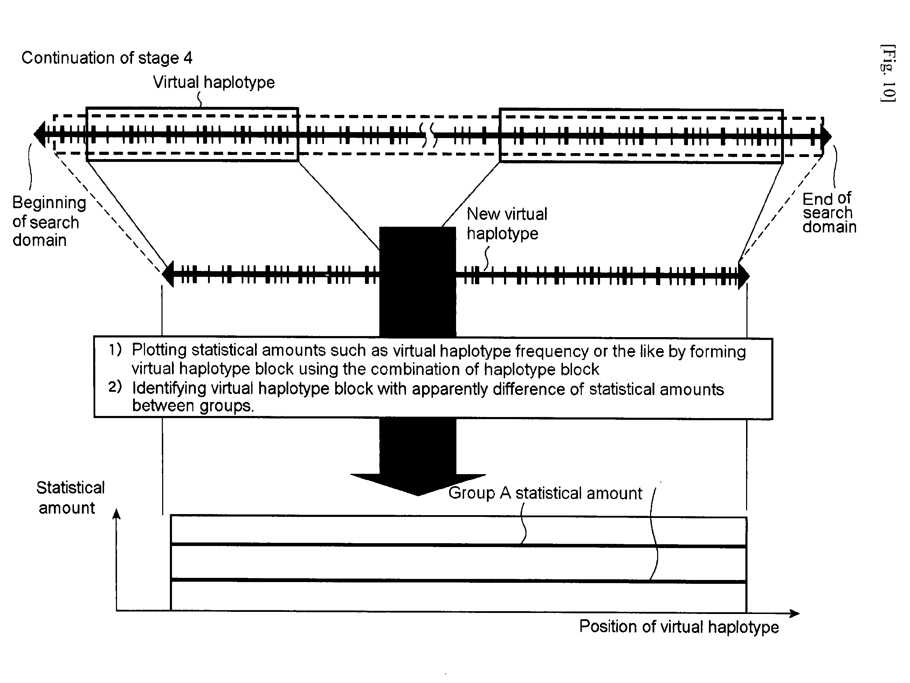

[Fig.10] Fig. 10 shows an example of extracting the combination of haplotype block which has an apparent difference between two groups from the virtual block constructed in Fig. 9.
[Fig.11] Fig. 11 shows an example of gene polymorphism data obtained by typing with a wet process in stage 4 of Fig. 1.
[Fig.12] Fig. 12 shows an example of calculating the haplotype frequency when not specifying the sample phase of ID01 in Fig. 11.
[Fig.13] Fig. 13 shows an example of calculating the haplotype frequency from gene polymorphism data in Fig. 11.
[Fig.14] Fig. 14 shows an example of calculating the haplotype frequency from gene polymorphism data in Fig. 11.
[Fig.15] Fig. 13 shows an example of calculating the maximum likelihood origin haplotype and its haplotype frequency in stage 7 of Fig. 1.
[Fig.16] Fig. 16 is a brief summary showing an example of a block drawing indicating the system of specifying 'relevant genome domain' of an embodiment of the present invention.
[Fig.17] Fig. 17 is a drawing showing an example of a process flowchart indicating the conventional relevant gene function analysis.

### [Explanation of code]

11 Virtual block constructing section
12 Haplotype block specifying section
13 Frequency calculating section
14 Haplotype block with difference specifying section
15 Relevant genome identifying section

## Claims

1. A method of systematic analysis of relevant gene identifying relevant genome domain of a single or a plurality of relevant gene / relevant haplotype or the like related to disease susceptibility, drug responsiveness, or the like (abbreviation afterward as 'relevant genome domain') from the information about a whole genome domain or a discontinuous genome domain which can be included in a part of function to be desired to analyze is elucidated or is not all estimated (abbreviation afterward 'search domain') and comprising:
a first step of constructing a virtual block from said search domain;
a second step of specifying haplotype block by scanning said virtual block using virtual haplotype;
a third step of calculating haplotype frequency by haplotype analysis, associated analysis, or the like in said haplotype block;
a fourth step of specifying haplotype block which has an apparent difference by said haplotype analysis, associated analysis, or the like; and
a fifth step of identifying said relevant genome domain from said haplotype block.

2. The method of systematic analysis of relevant gene according to claim 1
wherein said first step comprises a step of selecting a known marker for every haplotype block when the marker representing the each haplotype block is known, and making the continuous virtual block by connecting over the marker.

3. The method of systematic analysis of relevant gene according to claim 1
wherein said first, second, third, fourth, and fifth step comprises a step of determining the gene polymorphism marker to specify said relevant genome domain and gradually (including one step) narrowing down said relevant genome domain by repeating all the steps or a part of step.

4. The method of systematic analysis of relevant gene according to claim 1
wherein said second step comprises a step of determining a single or a plurality of haplotype block which is linkage disequilibrium (or chains) state to said relevant genome domain by using statistical analysis such as virtual haplotype analysis or the like

5. The method of systematic analysis of relevant gene according to claim 1
wherein said third step comprises a step of calculating the maximum likelihood origin haplotype and the frequency thereof by using the combination of associated analysis, haplotype analysis, and the like.

6. The method of systematic analysis of relevant gene according to claim 1
wherein said fourth step comprises a step of identifying said haplotype block including haplotype which has an apparent difference by said associated analysis.

7. The method of systematic analysis of relevant gene according to claim 1
wherein said second step comprises a step of determining the border of said haplotype block by using statistical data such as the number of combination of virtual haplotype, entropy value, the number of said maximum likelihood origin haplotype, linkage disequilibrium value, or the like.

8. The method of systematic analysis of relevant gene according to claim 1
wherein said third step comprises a step of determining a group of said maximum likelihood origin haplotype by using EM algorithm, MCMC method, or the like.

9. The method of systematic analysis of relevant gene according to claim 1
wherein said fourth step comprises a step of comparing a calculated statistical amount by associated analysis or the like with a preset or measured reference statistical amount, and when there is significant deviation between said statistical amount and said reference statistical amount that exceeds a preset threshold, determining said relevant genome domain is included in the domain (haplotype block) corresponding to the deviated position that exceeds said threshold value.

10. The method of systematic analysis of relevant gene according to claim 1
wherein said fifth step comprises a step of further detailed scanning / analyzing haplotype block or haplotype which has an apparent difference by said associated analysis or the like by using sequencing or the like and determining said relevant genome domain.

11. The method of systematic analysis of relevant gene according to claim 1
wherein said fifth step comprises a step of selecting typing gene polymorphism marker having an interval that is at least shorter than the length of haplotype block in said search genome domain and that is as uniform as possible.

12. The method of systematic analysis of relevant gene according to claim 1
wherein said fifth step comprises a step of selecting the gene polymorphism marker with easy typing, which the gene polymorphism marker for typing in a group history expression is at least older than the phenotype which consider to check the relationship (SNP whose minor allele frequency is not so small number when the gene polymorphism is SNP) without limiting the cDNA domain or exon domain.

13. The method of systematic analysis of relevant gene according to claim 1
wherein said first, second, third, fourth, and fifth step comprises a step of determining the most preferred relevant haplotype block or relevant haplotype by changing the selecting method for virtual haplotype (length or the like).

14. A computer program that can be read by a computer that can execute the processing of the method of specifying SNP according to any one of the claims 1 thru 13 wherein all of the steps of any one of the claims 1 thru 13 are coded.

15. A system of analysis of discontinuous domain identifying relevant genome domain of a single or a plurality of relevant gene / relevant haplotype or the like related to disease susceptibility, drug responsiveness, or the like from the information about the search domain as a whole genome domain or a discontinuous genome domain which can be included in a part of function to be desired to analyze is elucidated or is not all estimated and comprising:
a means of constructing a virtual block from said search domain;
a first means of specifying haplotype block by scanning said virtual block by using virtual haplotype;
a means of calculating haplotype frequency by haplotype analysis, associated analysis, or the like in said haplotype block;
a second means of specifying haplotype block which has an apparent difference by said haplotype analysis, associated analysis, or the like; and
a means of identifying said relevant genome domain from said haplotype block.
